# FASCICULE DE BREVET EUROPEEN

(11) **EP 2 737 925 B1**
(45) Date de publication et mention de la délivrance du brevet: **17.06.2015**
(21) Numéro de dépôt: 13185651.0
(22) Date de dépôt: 24.09.2013
(51) Int. Cl.: A61N 1/362, A61N 1/368, A61N 1/37, A61N 1/365, A61B 5/024

(54) **Prothèse cardiaque implantable active avec détection de l'activité mécanique auriculaire**
Aktive implantierbare Herzprothese mit Erfassung der aurikularen mechanischen Aktivität
Active implantable cardiac prosthesis with detection of atrial mechanical activity

(30) Priorité: 29.11.2012 FR 1261424
(43) Date de publication de la demande: 04.06.2014
(73) Titulaire: Sorin CRM SAS, 92140 Clamart Cedex (FR)
(72) Inventeur: Anselmi, Francesca, 92120 Montrouge (FR)
(74) Mandataire: Dupuis-Latour, Dominique

(56) Documents cités:
- EP-A1- 2 092 885
- EP-A1- 2 189 180
- EP-A1- 2 471 575
- US-A1- 2007 179 542

## Description

L'invention concerne les "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation et/ou de défibrillation en cas de trouble du rythme détecté par le dispositif. L'invention concerne plus particulièrement le diagnostic des troubles de la contraction auriculaire.

Ces troubles apparaissent le plus souvent comme séquelles d'un épisode de fibrillation auriculaire (FA ou AF), c'est-à-dire d'une arythmie caractérisée par des fréquences anormalement élevées du rythme auriculaire.

Il peuvent également résulter d'autres pathologies telles qu'ischémie auriculaire, ou oreillette dilatée par une sarcoïdose ou une fibrose.

Après un épisode de FA, il arrive en effet que la contraction du myocarde au niveau de l'oreillette soit déficiente, voire absente, malgré la présence du point de vue électrique d'une onde de dépolarisation observable, spontanée ou stimulée.

Une telle atonie du muscle de l'oreillette entraine une moindre contribution de celle-ci au remplissage du ventricule, avec de ce fait une dégradation très nette des performances hémodynamiques.

De plus, dans une telle situation il n'est pas possible d'optimiser un fonctionnement de type double chambre, c'est-à-dire avec détection auriculaire et ventriculaire et stimulation ventriculaire. Dans ce mode de fonctionnement, le dispositif déclenche si nécessaire une stimulation ventriculaire après écoulement d'un délai auriculo-ventriculaire (DAV ou AVD) prédéterminé, compté à partir de la détection d'un évènement auriculaire spontané ou stimulé. Ce DAV est normalement ajusté de manière à optimiser globalement le fonctionnement du coeur du point de vue hémodynamique. En cas d'atonie auriculaire, l'activité électrique normale entrainera l'application du DAV, mais il sera impossible d'optimiser ce délai du fait de l'ignorance du comportement réel de l'oreillette.

Or, à l'exception d'une exploration par échocardiographie, il n'est pas possible de déterminer si après un épisode de FA un patient a bien récupéré une activité auriculaire mécanique normale et le moment où cette récupération est intervenue, ou au contraire s'il présente une atonie auriculaire, c'est-à-dire une contraction de l'oreillette absente ou déficiente en dépit d'une activité électrique observable (rythme sinusal).

Le but de l'invention est de proposer un dispositif capable d'évaluer l'activité mécanique de l'oreillette, notamment de manière à avérer après un épisode de FA le retour à une activité auriculaire normale - ou bien, au contraire, diagnostiquer la présence d'une atonie auriculaire.

Le principe de l'invention consiste, pour évaluer l'activité mécanique de l'oreillette, à mesurer dans un signal d'accélération endocardiaque (EA) un paramètre d'une composante liée à l'activité mécanique du ventricule, et à opérer une modulation du DAV : s'il résulte de cette modulation une variation significative du paramètre en question, alors ceci est censé refléter une activité mécanique correcte de l'oreillette ; inversement, l'absence de variation significative du paramètre peut indiquer une activité auriculaire absente ou dégradée.

Grâce à une telle analyse, un dispositif implanté pourra établir de façon automatique l'état de l'activité auriculaire du patient et permettra, en particulier, de déterminer le moment où le patient aura recouvré une activité auriculaire mécanique normale après un épisode de FA.

De plus, en cas d'absence d'activité mécanique auriculaire avérée, il sera possible de désactiver des algorithmes qui seraient de toute façon inefficaces en l'absence d'une telle activité, par exemple des algorithmes d'optimisation automatique du DAV.

Le US 2007/0179542 A1 décrit un dispositif comprenant des moyens d'ajustement du DAV permettant d'optimiser dynamiquement l'état hémodynamique du patient. Le dispositif est en outre capable de détecter la présence ou non d'une fibrillation atriale, d'une tachycardie atriale ou autre épisode pathologique de même nature sur l'oreillette, et d'inhiber l'ajustement du DAV pendant la durée d'un tel épisode avéré. En effet, un calcul d'ajustement serait très fortement biaisé par l'état pathologique du patient et pourrait engendrer chez celui-ci des effets délétères.

L'objet de l'invention n'est pas, comme dans le US 2007/0179542 A1, de prendre une action en fonction de la présence ou non, d'un épisode de fibrillation atriale. L'objet de l'invention est - une fois que la fibrillation a pris fin - d'évaluer la qualité (à savoir : normale, atone, voire quasi-absente) de la contraction auriculaire en rythme sinusal, c'est-à-dire la qualité de l'activité mécanique de l'oreillette lorsque celle-ci présente une activité électrique observable.

À cet effet, l'invention propose un dispositif médical implantable actif de type prothèse cardiaque de stimulation, resynchronisation et/ou défibrillation, comportant de manière en elle-même connue, notamment d'après le US 2007/0179542 A1 précité :
- des moyens de détection d'évènements auriculaires et ventriculaires ;
- des moyens de stimulation ventriculaire ;
- des moyens pour appliquer aux moyens de stimulation un délai auriculo-ventriculaire, DAV, compté à partir de la détection d'un évènement auriculaire spontané ou stimulé et à l'issue duquel une stimulation ventriculaire est délivrée en l'absence de détection d'un évènement ventriculaire spontané correspondant ;
- un capteur d'accélération apte à délivrer un signal d'accélération endocardiaque, EA, représentatif des mouvements produits par les contractions cycliques du myocarde ;
- des moyens d'analyse du signal EA, aptes à dériver de ce signal EA une valeur d'un paramètre ventriculaire EA non temporel représentatif d'une composante EA1 du signal correspondant au premier pic EA associé à la contraction ventriculaire isovolumique ;
- des moyens de balayage, aptes à faire varier de façon contrôlée le DAV dans une plage comportant une pluralité de valeurs de DAV ; et
- des moyens quantificateurs, pour évaluer un degré de variation dudit paramètre ventriculaire EA en fonction de ladite pluralité de valeurs du DAV.

De façon caractéristique de l'invention, le dispositif comprend en outre des moyens de détection d'activité auriculaire, aptes à discriminer entre : i) une activité mécanique auriculaire normale et ii) une activité mécanique auriculaire absente ou déficiente en dépit d'une activité électrique observable en rythme sinusal, en fonction dudit degré de variation évalué par les moyens quantificateurs.

Le paramètre ventriculaire EA non temporel peut notamment être l'amplitude entre extrema du signal EA de la composante EA1, ou l'énergie de la composante EA1.

Dans un premier mode de mise en oeuvre de l'invention :
- ladite plage de valeurs de DAV comprend une première plage avec une pluralité de DAV courts et une seconde plage, distincte de la première, avec une pluralité de DAV longs ;
- les moyens quantificateurs sont aptes à évaluer ledit degré de variation par calcul d'une différence entre les valeurs du paramètre ventriculaire EA relevées pour les DAV de la première plage et celles relevées pour la seconde plage ; et
- les moyens de détection d'activité auriculaire sont aptes à discriminer entre une activité mécanique auriculaire normale lorsque ladite différence est supérieure à un seuil prédéterminé, et une activité mécanique auriculaire absente ou déficiente dans le cas contraire.

Dans un deuxième mode de mise en oeuvre de l'invention :
- les moyens quantificateurs sont aptes à évaluer ledit degré de variation par calcul d'un écart-type des valeurs du paramètre ventriculaire EA relevées pour les divers DAV de ladite plage de valeurs ; et
- les moyens de détection d'activité auriculaire sont aptes à discriminer entre une activité mécanique auriculaire normale lorsque ledit écart-type est supérieur à un seuil prédéterminé, et une activité mécanique auriculaire absente ou déficiente dans le cas contraire.

Dans un troisième mode de mise en oeuvre de l'invention :
- les moyens quantificateurs sont aptes à évaluer ledit degré de variation par modélisation de la caractéristique sigmoïde de variation du paramètre ventriculaire EA en fonction des DAV de ladite plage de valeurs, avec deux plateaux de part et d'autre d'une partie centrale de transition ; et
- les moyens de détection d'activité auriculaire sont aptes à discriminer entre une activité mécanique auriculaire normale lorsque la différence absolue de niveau des deux plateaux est supérieure à un seuil prédéterminé, et une activité mécanique auriculaire absente ou déficiente dans le cas contraire.

La technique de l'invention peut notamment servir à mémoriser un indice fonction dudit degré de variation du paramètre ventriculaire EA en fonction de la pluralité de valeurs du DAV, pour constituer un historique de l'évolution dudit indice au cours du temps.

En particulier, lorsque le dispositif comprend en outre des moyens de stimulation auriculaire, il est possible de : mettre en oeuvre lesdits moyens de détection d'activité auriculaire en présence d'au moins un évènement auriculaire spontané et d'au moins un évènement auriculaire stimulé ; comparer les valeurs respectives mémorisées dudit indice ; et dériver de cette comparaison un indicateur d'alerte en cas de discordance entre les indices respectifs comparés. Ainsi qu'éventuellement, en cas de survenue d'un épisode de fibrillation auriculaire, à : comparer les valeurs mémorisées dudit indice respectivement antérieures et postérieures à l'épisode de fibrillation auriculaire ; et dériver de cette comparaison un indicateur de récupération, ou d'absence de récupération, après fibrillation auriculaire.

On va maintenant décrire un exemple de mise en oeuvre du dispositif de l'invention, en référence aux dessins annexés où les mêmes références numériques désignent d'une figure à l'autre des éléments identiques ou fonctionnellement semblables.
La Figure 1 est une série de trois chronogrammes illustrant différents signaux caractéristiques qu'il est possible de recueillir au cours d'un cycle cardiaque.
La Figure 2 montre de façon plus détaillée l'allure du signal d'accélération endocardiaque au cours d'un cycle donné.
Les Figures 3a à 3c illustrent la forme classique d'une caractéristique de variation de l'amplitude du premier pic du signal EA en fonction du DAV, pour un sujet sain (Figure 3a) et avec les modifications que cette caractéristique peut présenter dans le cas d'une absence d'activité mécanique auriculaire (Figure 3b) ou en cas d'atonie auriculaire (Figure 3c).
Les Figures 4a et 4b illustrent une première technique d'analyse de la caractéristique de la Figure 3a pour discriminer entre activité auriculaire normale et anormale ou absente.
Les Figures 5a et 5b sont homologues des précédentes, pour une seconde technique d'analyse.
Les Figures 6a et 6b sont homologues des précédentes, pour une troisième technique d'analyse.
La Figure 7 illustre la manière d'opérer un suivi sur plusieurs semaines de l'activité auriculaire, dans le cas où celle-ci est considérée comme normale.
La Figure 8 est homologue de la Figure 7, dans le cas d'un épisode de fibrillation auriculaire laissant subsister pendant plusieurs semaines une activité auriculaire mécanique déficiente, jusqu'au moment où cette activité peut être considérée comme redevenue normale.
La Figure 9 est un organigramme décrivant de façon schématique l'enchainement au cours du temps des différentes étapes de suivi et de diagnostic de l'activité auriculaire.

On va maintenant décrire un exemple de réalisation de l'invention, en référence aux dessins annexés.

L'invention peut notamment être appliquée aux dispositifs implantables tels que ceux de la famille *Paradym* (en particulier le *Paradym RF SonR CRT-D*) produits et commercialisés par Sorin CRM, Clamart, France.

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre certaines des fonctions de l'invention qui seront décrites ci-dessous (optimisation des paramètres et suivi de l'état du patient). L'adaptation de ces appareils à la mise en oeuvre des fonctions de l'invention est à la portée de l'homme du métier, et elle ne sera pas décrite en détail.

La technique de l'invention est basée sur l'analyse de l'accélération endocardiaque (ci-après désignée "EA"), qui est un paramètre qui reflète très précisément et en temps réel les phénomènes concourant au fonctionnement mécanique du myocarde et qui peut être mesuré par un accéléromètre couplé au muscle cardiaque, comme cela est décrit par exemple dans le EP 0 515 319 A1 (Sorin Biomedica Cardio SpA). Ce document enseigne la manière de recueillir un signal EA au moyen d'une sonde endocavitaire pourvue d'une électrode distale de stimulation implantée dans l'oreillette ou le ventricule et intégrant un microaccéléromètre permettant de mesurer l'accélération endocardiaque.

Un tel capteur peut être par exemple disposé sur une sonde endocavitaire aboutissant au fond du ventricule, pourvue à cet endroit d'un capteur d'accélération. Il peut s'agir également d'une sonde auriculaire pourvue à son extrémité disposée contre la paroi de l'oreillette droite d'un capteur d'accélération. On notera toutefois que, bien que dans la présente description on fasse principalement référence à l'analyse d'un signal EA délivré par un capteur placé sur une sonde endocavitaire, l'invention est également applicable à une analyse opérée à partir d'un signal EA délivré par d'autres types de capteurs implantés, tels qu'un capteur de mouvement d'une paroi du myocarde, un capteur épicardique ou un accéléromètre placé dans le boitier d'un implant. L'invention est également applicable à l'analyse d'un signal EA externe recueilli de manière non invasive, par exemple issu d'un capteur fixé sur la poitrine du patient au niveau du sternum.

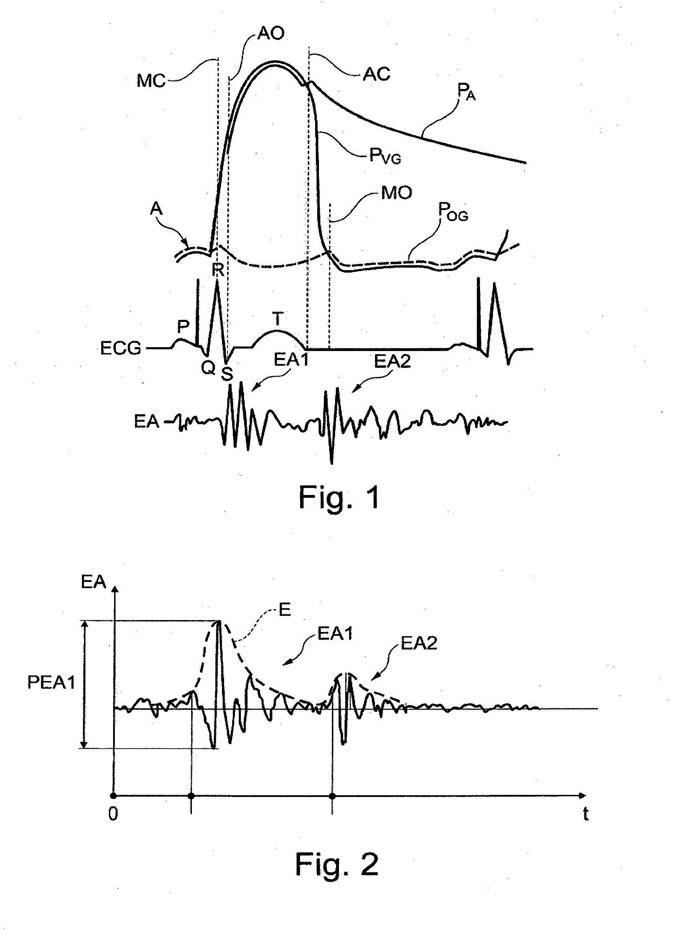

La Figure 1 illustre les différents signaux caractérisant l'activité du coeur au cours d'un cycle cardiaque, avec :
- le profil des pressions intracardiaques P_{A}, P_{VG} et P_{OG} : la caractéristique P_{A} illustre les variations de la pression aortique, P_{VG} celles du ventricule gauche et P_{OG} celles de l'oreillette gauche. Ces variations passent par les différentes phases suivantes : A contraction de l'oreillette gauche, MC fermeture de la valve mitrale, AO ouverture de la valve aortique, AC fermeture de la valve aortique, MO ouverture de la valve mitrale ;
- un relevé d'électrocardiogramme de surface ECG, avec successivement : l'onde P correspondant à la dépolarisation des oreillettes, le complexe QRS correspondant à la dépolarisation des ventricules, et l'onde T de repolarisation ventriculaire ; et
- les variations du signal EA d'accélération endocardiaque recueilli, qui forme deux composantes principales EA1 et EA2 au cours d'un cycle cardiaque donné, correspondant aux deux bruits majeurs du coeur (sons S1 et S2 du phonocardiogramme) qu'il est possible de reconnaitre dans chaque cycle cardiaque.

Sur la Figure 2, on a illustré plus précisément les variations de ce signal EA au cours d'un cycle cardiaque, montrant :
- la composante EA1, qui débute à la suite du complexe QRS et qui est engendrée par une combinaison de la fermeture des valves atrioventriculaires (valve mitrale et valve tricuspide), de l'ouverture des valves semilunaires (valve aortique et valve pulmonaire) et de la contraction du ventricule gauche. Les variations d'amplitude de cette composante EA1 sont étroitement liées aux variations de la pression dans le ventricule, l'amplitude maximale crête-à-crête PEA1 étant plus précisément corrélée au maximum positif de la variation de pression dP/dt dans le ventricule gauche ; et
- la composante EA2, qui survient pendant la phase de relaxation ventriculaire isovolumique. Elle accompagne la fin de la systole ventriculaire et est principalement produite par la fermeture des valves aortique et pulmonaire.

Pour la mise en oeuvre de l'invention, il convient d'extraire du signal EA, et plus précisément de la composante EA1, un paramètre non temporel caractéristique de l'ampleur de cette composante EA1.

Ce paramètre peut être notamment l'amplitude PEA1 du premier pic d'accélération endocardiaque, c'est-à-dire la valeur maximale crête-à-crête séparant les deux extrema, positif et négatif, de la composante EA1 du signal d'accélération.

C'est ce paramètre PEA1 qui sera utilisé dans la suite de la description comme paramètre non temporel significatif, mais ce choix n'est cependant aucunement limitatif, et d'autres paramètres représentatifs de l'ampleur de la composante EA1 peuvent être utilisés.

En particulier, il est possible d'utiliser comme paramètre non temporel significatif l'énergie du signal EA contenue dans tout ou partie de la composante EA1. Cette énergie est donnée par la valeur efficace (racine carrée de la moyenne des carrés, ou RMS) d'une série d'échantillons considérés pendant une fenêtre de durée prédéterminée de la composante EA1. Ce paramètre d'énergie est illustré sur la Figure 2 par l'enveloppe E du signal obtenu en élevant au carré la valeur des échantillons du signal EA. L'analyse du signal EA est déterminée de préférence avec moyennage sur plusieurs cycles, typiquement trois à cinq cycles, en appliquant une technique telle que celle exposée dans le EP 2 092 885 A1 (ELA Medical), technique qui permet notamment d'éliminer les variations cycle à cycle en recalant dans le temps les composantes successives avant de les moyenner.

Essentiellement, cette technique consiste à effectuer un prétraitement du signal EA recueilli en continu, avec :
- découpage du signal EA en sous-signaux correspondant chacun à la durée d'un cycle cardiaque et repérés par des marqueurs de début de cycle (origine des temps) permettant de réaliser ce découpage. Les marqueurs temporels de début de cycle peuvent être fournis par le dispositif implanté qui, selon le mode de fonctionnement, garde en mémoire les instants de la stimulation V ou bien les instants de détection de l'onde R ;
- segmentation de chacun de ces sous-signaux de manière à individualiser la composante EA1 dans une fenêtre temporelle donnée ;
- pour la composante courante EA1 ainsi isolée sur un cycle, recherche d'un pic d'intercorrélation par rapport aux composantes EA1 des autres cycles recueillis ;
- calcul d'un décalage temporel correspondant ; et
- application du décalage temporel ainsi calculé à la composante courante, de manière à aligner celle-ci par rapport aux autres.

Les traitements d'analyse du signal EA pourront être ensuite exécutés sur les composantes EA1 successives, avec élimination du biais de la variabilité cycle-à-cycle grâce à ce prétraitement.

Le principe de base de l'invention consiste à moduler le délai auriculo-ventriculaire (DAV ou AVD) et à examiner les variations produites par cette modulation sur le paramètre caractéristique non temporel du signal EA, dans cet exemple l'amplitude PEA1.

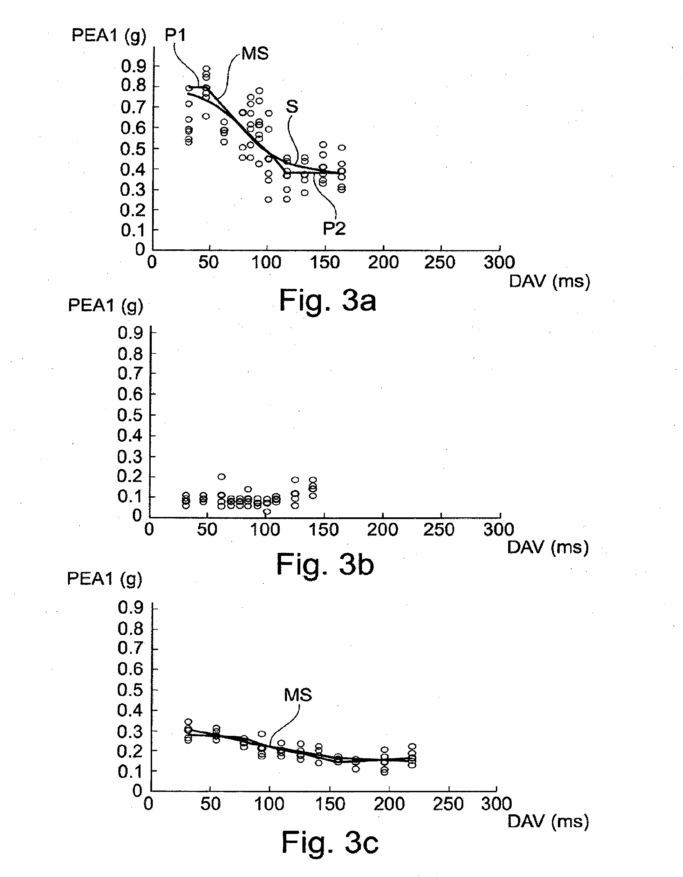

La variation de cette amplitude avec le DAV est bien connue, et elle est normalement associée à une caractéristique de forme sigmoïde S, illustrée Figure 3a.

Sur cette figure et sur les suivantes, les cercles en trait fin indiquent les différentes valeurs relevées au cours de cycles cardiaques successifs pour une même valeur de DAV appliquée, et les cercles en trait for correspondent à la valeur de ces différentes mesures moyennée pour un même DAV.

L'amplitude PEA1 décroit lorsqu'augmente le DAV, et la caractéristique sigmoïde peut être modélisée sous une forme simplifiée MS avec deux plateaux P1, P2 correspondant respectivement aux DAV courts et aux DAV longs, ces plateaux étant séparés par une partie centrale de transition de pente négative.

L'invention repose sur la constatation que les variations de la caractéristique EA1 en fonction du DAV, notamment les variations de l'amplitude PEA1, sont dues entre autres facteurs à la contribution différente de l'oreillette au remplissage du ventricule : un DAV court maximise ce remplissage, conduisant à un pic de signal EA très marqué, tandis qu'un DAV long réduit l'amplitude et l'énergie de la composante EA1.

Lorsque l'activité mécanique auriculaire est absente, typiquement après un épisode de fibrillation auriculaire (FA), l'oreillette ne contribue en aucune façon au remplissage du ventricule, et une variation du DAV ne produit aucune variation significative de la composante EA1.

Cette situation est illustrée sur la Figure 3b, où l'on peut voir que le niveau de l'amplitude PEA1 est sensiblement le même quel que soit le DAV appliqué, court ou long.

Dans le cas d'une contraction auriculaire présente, mais déficiente (atonie auriculaire) l'amplitude PEA1 varie légèrement avec le DAV (situation illustrée Figure 3c), mais beaucoup moins que dans le cas d'une contraction auriculaire complète (Figure 3a).

Différentes techniques peuvent être employées pour analyser la caractéristique PEA1/DAV et discriminer entre activité auriculaire normale et activité auriculaire absente ou déficiente.

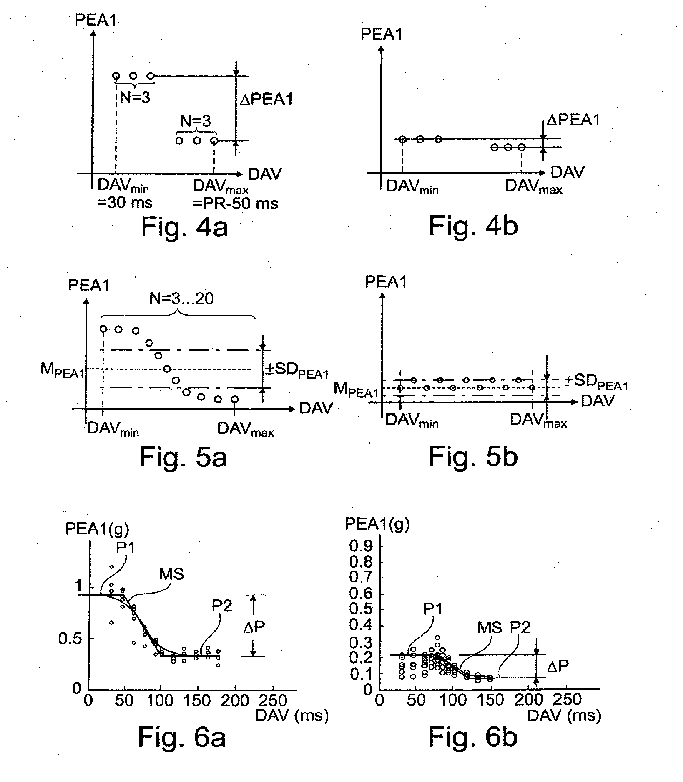

*Une première technique*, illustrée en référence aux Figures 4a et 4b, consiste à appliquer un certain nombre N de valeurs de DAV de test courts, puis autant de valeurs de DAV de test longs, et à mesurer l'amplitude PEA1 pour chacun des DAV appliqués.

Les DAV de test sont choisis dans une plage bornée par exemple par des valeurs minimale DAVₘᵢₙ = 32 ms pour la valeur la plus courte et maximale DAVₘₐₓ = PR-50 ms pour le DAV le plus long (PR étant l'intervalle entre les dépolarisations auriculaire et ventriculaire et 50 ms correspondant à une marge de sécurité prédéterminée). Le nombre N qui définit le nombre de DAV longs et courts à tester peut être compris par exemple entre 3 et 20. Les différents DAV testés le sont avec un pas fixe, par exemple 15 ms entre deux DAV consécutifs.

Pour chaque DAV testé, l'amplitude PEA1 est mesurée et moyennée sur un certain nombre de cycles, par exemple six cycles cardiaques ou plus. Pour déterminer le niveau d'activité auriculaire, une différence est calculée entre les moyennes des valeurs de PEA1 mesurées pour les N DAV courts, et celles mesurées pour les N DAV longs : si cette différence absolue est inférieure à un seuil donné programmable, on considère alors qu'il y a absence d'activité auriculaire mécanique normale (situation de la Figure 4b).

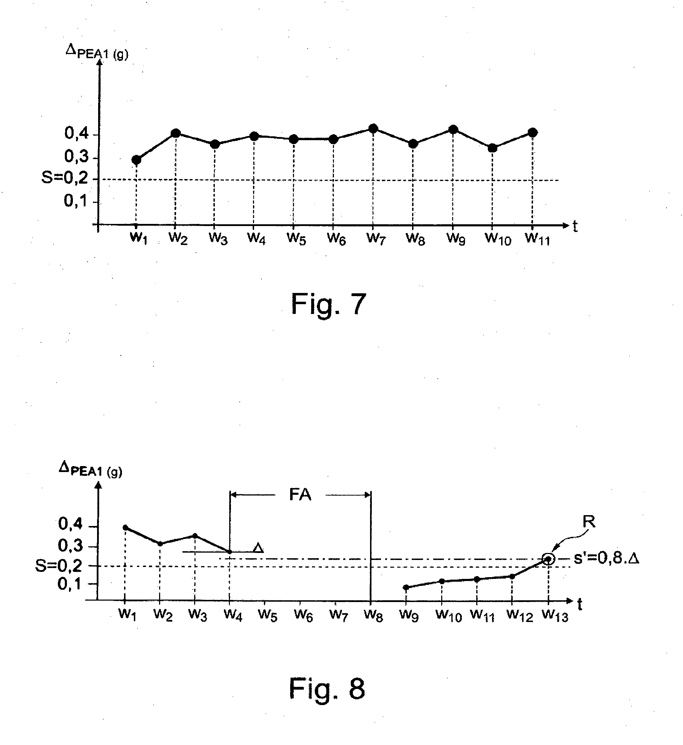

La valeur de la différence calculée est avantageusement conservée en mémoire, de manière à constituer un historique qui permettra d'assurer un suivi sur le long terme de l'activité mécanique auriculaire, comme on l'expliquera plus loin en référence aux Figures 7 et 8.

*Une deuxième technique,* illustrée en référence aux Figures 5a et 5b, consiste à moduler le DAV de façon régulière par un balayage entre les valeurs DAVₘᵢₙ et DAVₘₐₓ, de manière à obtenir un nombre N, typiquement compris entre 4 et 20, de mesures successives entre valeurs de DAV équidistantes. Pour chaque DAV testé, l'amplitude PEA1 est mesurée et moyennée sur plusieurs cycles, typiquement au moins six cycles cardiaques.

L'écart-type SD_{PEA1} est alors calculé à partir de toutes les valeurs moyennées de PEA1 : si cet écart-type est inférieur à un seuil donné, on considère alors qu'il y a absence d'activité auriculaire mécanique normale (situation de la Figure 5b).

Ici encore, la valeur de l'écart-type calculé est conservée en mémoire, pour assurer un suivi sur le long terme de l'activité mécanique auriculaire du patient.

*Une troisième technique*, illustrée en référence aux Figures 6a et 6b, consiste, en variante de la précédente, opérer une modélisation MS de la caractéristique obtenue après balayage de la plage des DAV possibles entre DAVₘᵢₙ et DAVₘₐₓ.

Comme on l'a indiqué plus haut, la caractéristique modélisée présente deux plateaux P1 pour les DAV les plus faibles et P2 pour les DAV les plus forts, typiques d'une forme sigmoïde. L'activité auriculaire est estimée en calculant la différence absolue ΔP entre les deux plateaux P1 et P2 de l'approximation sigmoïde : si cette différence est inférieure à un seuil donné, on considère alors qu'il y a absence d'activité auriculaire mécanique normale (situation de la Figure 6b).

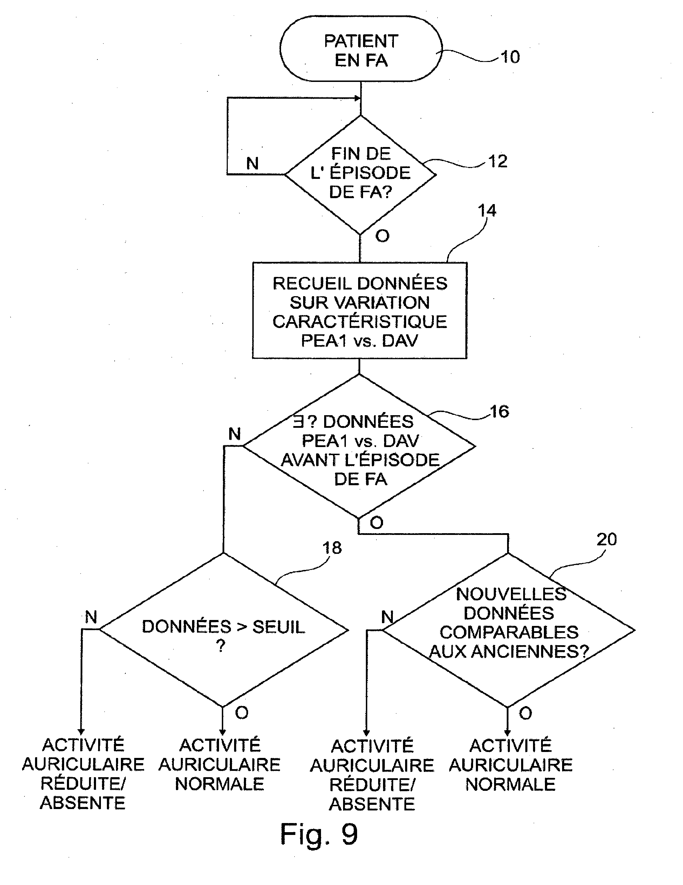

Les Figures 7 et 8, et l'organigramme de la Figure 9, illustrent la possibilité d'utiliser le test d'activité mécanique auriculaire que l'on vient d'exposer pour assurer un suivi sur le long terme de l'état clinique du patient.

Ce test peut être déclenché de manière périodique, par exemple de façon hebdomadaire (ou plus fréquemment) afin de déterminer le statut de l'activité auriculaire. Ce test est exécuté à la fois pour des dépolarisations auriculaires spontanées et stimulées. Dans le cas d'une activité auriculaire stimulée, on prendra soin de se placer dans des conditions où une capture auriculaire est certaine ; pour cela, on ajustera une amplitude de stimulation à un niveau élevé pendant le test, ou bien on vérifiera au préalable la présence d'une capture auriculaire effective.

Pour le test, on module le DAV afin d'évaluer un degré de variation de la caractéristique EA1 en fonction du DAV, selon l'une des techniques exposées plus haut. Par exemple, on mémorise la différence Δ_{PEA1} calculée entre les valeurs d'amplitude PEA1 relevées pour les DAV courts et celles relevées pour les DAV longs : si cette valeur Δ_{PEA1} est supérieure à un seuil S donné, on considère que l'activité auriculaire est présente et normale, dans le cas contraire on considère qu'elle est absente ou déficiente (atonie auriculaire).

En tout état de cause, le dispositif compare les valeurs respectives mémorisées de la valeur Δ_{PEA1} en présence d'un évènement auriculaire spontané et d'un évènement auriculaire stimulé, et génère une alerte en cas de discordance entre les valeurs comparées.

La Figure 7 illustre un exemple dans lequel la valeur Δ_{PEA1} est évaluée à intervalles hebdomadaires W1, W2, W3... et mémorisée de manière à constituer un historique sur le long terme de l'activité mécanique auriculaire globale du patient, sur cette figure une activité mécanique auriculaire normale.

La Figure 8 illustre un exemple montrant un épisode de fibrillation auriculaire FA, laissant subsister pendant plusieurs semaines une activité auriculaire mécanique déficiente.

La fin de cet épisode (entre les périodes W4 et W8) peut être détectée par un retour du rythme sinusal, et le test d'activité mécanique auriculaire selon l'invention est alors effectué. Sur l'exemple de la figure, ce test indique aux périodes W9 à W12 une activité auriculaire déficiente, le degré de variation PEA1/DAV étant inférieur à un seuil fixé. On peut considérer que le patient a recouvré une activité auriculaire normale lorsque Δ_{PEA1} remonte au-dessus de ce seuil, qui est par exemple un seuil S' = 80 % de la valeur Δ prise par le paramètre de variation Δ_{PEA1} avant le début de l'épisode FA.

La Figure 9 illustre schématiquement les étapes successives de l'algorithme permettant de discriminer entre les divers états possibles d'activité mécanique auriculaire après un épisode de fibrillation auriculaire.

Pour un patient en fibrillation auriculaire (bloc 10), le dispositif attend la fin de cet épisode (bloc 12), détecté par le retour à un rythme sinusal normal et stable, révélé par l'analyse de l'activité électrique des signaux de dépolarisation détectés au niveau de l'oreillette.

Il devient alors possible d'effectuer un test pour évaluer le degré de variation de l'amplitude PEA1 en fonction du DAV (bloc 14).

Si des résultats de test avaient été obtenus et mémorisés avant l'épisode de FA (bloc 16), alors l'algorithme compare les nouvelles données aux anciennes (bloc 20). En cas de données comparables (par exemple si le paramètre de variation Δ_{PEA1} est d'au moins 80 % de la valeur qu'il avait avant l'épisode de FA), alors on considère que l'activité auriculaire est redevenue normale ; dans la négative, on considère que l'activité est insuffisante ou absente.

En l'absence de données antérieures à l'épisode de FA, l'algorithme se contente de comparer à un seuil le résultat du test (bloc 18) et à discriminer en fonction de ce résultat entre activité auriculaire mécanique normale ou bien réduite ou absente.

L'analyse de l'activité mécanique auriculaire permet également d'activer ou de désactiver sélectivement divers algorithmes tels que *overdriving* auriculaire, optimisation automatique du DAV, etc. en fonction du résultat du test.

## Revendications

1. Un dispositif médical implantable actif de type prothèse cardiaque de stimulation, resynchronisation et/ou défibrillation, comportant :
- des moyens de détection d'évènements auriculaires et ventriculaires ;
- des moyens de stimulation ventriculaire ;
- des moyens pour appliquer aux moyens de stimulation un délai auriculo-ventriculaire, DAV, compté à partir de la détection d'un évènement auriculaire spontané ou stimulé et à l'issue duquel une stimulation ventriculaire est délivrée en l'absence de détection d'un évènement ventriculaire spontané correspondant ;
- un capteur d'accélération apte à délivrer un signal d'accélération endocardiaque, EA, représentatif des mouvements produits par les contractions cycliques du myocarde ;
- des moyens d'analyse du signal EA, aptes à dériver de ce signal EA une valeur d'un paramètre ventriculaire EA non temporel représentatif d'une composante EA1 du signal correspondant au premier pic EA associé à la contraction ventriculaire isovolumique ;
- des moyens de balayage, aptes à faire varier de façon contrôlée le DAV dans une plage comportant une pluralité de valeurs de DAV ; et
- des moyens quantificateurs, pour évaluer un degré de variation dudit paramètre ventriculaire EA en fonction de ladite pluralité de valeurs du DAV,
**caractérisé en ce qu'il** comprend en outre :
- des moyens de détection d'activité auriculaire, aptes à discriminer entre :
- une activité mécanique auriculaire normale et
- une activité mécanique auriculaire absente ou déficiente en dépit d'une activité électrique observable en rythme sinusal,
en fonction dudit degré de variation évalué par les moyens quantificateurs.

2. Le dispositif de la revendication 1, dans lequel ledit paramètre ventriculaire EA non temporel est l'amplitude entre extrema (PEA1) du signal EA de la composante EA1.

3. Le dispositif de la revendication 1, dans lequel ledit paramètre ventriculaire EA non temporel est l'énergie (E) de la composante EA1.

4. Le dispositif de la revendication 1, dans lequel :
ladite plage de valeurs de DAV comprend une première plage avec une pluralité de DAV courts et une seconde plage, distincte de la première, avec une pluralité de DAV longs ;
- les moyens quantificateurs sont aptes à évaluer ledit degré de variation par calcul d'une différence (Δ_{PEA1}) entre les valeurs du paramètre ventriculaire EA relevées pour les DAV de la première plage et celles relevées pour la seconde plage ; et
- les moyens de détection d'activité auriculaire sont aptes à discriminer entre une activité mécanique auriculaire normale lorsque ladite différence est supérieure à un seuil prédéterminé, et une activité mécanique auriculaire absente ou déficiente dans le cas contraire.

5. Le dispositif de la revendication 1, dans lequel :
- les moyens quantificateurs sont aptes à évaluer ledit degré de variation par calcul d'un écart-type (SD_{PEA1}) des valeurs du paramètre ventriculaire EA relevées pour les divers DAV de ladite plage de valeurs ; et
- les moyens de détection d'activité auriculaire sont aptes à discriminer entre une activité mécanique auriculaire normale lorsque ledit écart-type est supérieur à un seuil prédéterminé, et une activité mécanique auriculaire absente ou déficiente dans le cas contraire.

6. Le dispositif de la revendication 1, dans lequel :
- les moyens quantificateurs sont aptes à évaluer ledit degré de variation par modélisation de la caractéristique sigmoïde (MS) de variation du paramètre ventriculaire EA en fonction des DAV de ladite plage de valeurs, avec deux plateaux (P1, P2) de part et d'autre d'une partie centrale de transition ; et
- les moyens de détection d'activité auriculaire sont aptes à discriminer entre une activité mécanique auriculaire normale lorsque la différence absolue de niveau (AP) des deux plateaux est supérieure à un seuil prédéterminé, et une activité mécanique auriculaire absente ou déficiente dans le cas contraire.

7. Le dispositif de la revendication 1, comprenant en outre des moyens de mémorisation d'un indice fonction dudit degré de variation du paramètre ventriculaire EA en fonction de la pluralité de valeurs du DAV, et des moyens de constitution d'un historique de l'évolution dudit indice au cours du temps.

8. Le dispositif de la revendication 7, comprenant en outre :
- des moyens de stimulation auriculaire ; et
- des moyens aptes : à mettre en oeuvre lesdits moyens de détection d'activité auriculaire en présence d'au moins un évènement auriculaire spontané et d'au moins un évènement auriculaire stimulé ; à comparer les valeurs respectives mémorisées dudit indice ; et à dériver de cette comparaison un indicateur d'alerte en cas de discordance entre les indices respectifs comparés.

9. Le dispositif de la revendication 7, comprenant en outre des moyens aptes, en cas de survenue d'un épisode de fibrillation auriculaire (FA), à comparer les valeurs mémorisées dudit indice respectivement antérieures et postérieures à l'épisode de fibrillation auriculaire, et à dériver de cette comparaison un indicateur de récupération, ou d'absence de récupération, après fibrillation auriculaire.

## Patentansprüche

1. Aktive implantierbare medizinische Vorrichtung vom Typ Herzprothese zur Stimulation, Resynchronisation und/oder Defibrillation, umfassend:
- Mittel zum Erfassen von atrialen und ventrikulären Ereignissen;
- Mittel zur ventrikulären Stimulation;
- Mittel, um auf die Mittel zur Stimulation eine atrioventrikuläre Verzögerung, AVD, anzuwenden, die mit der Erfassung eines spontanen oder stimulierten atrialen Ereignisses beginnt, und an deren Ende, bei Abwesenheit eines entsprechenden spontanen, ventrikulären Ereignisses, eine ventrikuläre Stimulation bereitgestellt wird;
- einen Akzelerationssensor, der geeignet ist, ein Signal der endokardialen Akzeleration, EA, bereitzustellen, das für Bewegungen, die von den zyklischen Kontraktionen des Myokards erzeugt werden, repräsentativ ist;
- Mittel zur Analyse des EA-Signals, die geeignet sind, von diesem EA-Signal einen Wert eines nicht temporären, ventrikulären EA-Parameters abzuleiten, der für eine Komponente EA1 des Signals repräsentativ ist, die dem ersten Peak der EA entspricht, der mit der isovolumischen ventrikulären Kontraktion assoziiert ist;
- Mittel zum Abtasten, die geeignet sind, die AVD in einem Bereich, der eine Vielzahl von AVD-Werten umfasst, kontrolliert variieren zu lassen; und
- Quantifizierungsmittel zum Ermitteln eines Variationsgrads des ventrikulären EA-Parameters in Abhängigkeit von der Vielzahl von AVD-Werten,
**dadurch gekennzeichnet, dass** sie ferner umfasst:
- Mittel zum Erfassen der atrialen Aktivität, die geeignet sind, zwischen:
- einer normalen atrialen mechanischen Aktivität und
- einer atrialen mechanischen Aktivität, die trotz einer elektrischen Aktivität, die im Sinusrhythmus beobachtet werden kann, fehlt oder mangelhaft ist,
in Abhängigkeit von dem Variationsgrad, der von den Quantifizierungsmitteln ermittelt wurde, zu unterscheiden.

2. Vorrichtung nach Anspruch 1, wobei der nicht temporäre ventrikuläre EA-Parameter die Amplitude zwischen Extrema (PEA1) des EA-Signals der Komponente EA1 ist.

3. Vorrichtung nach Anspruch 1, wobei der nicht temporäre ventrikuläre EA-Parameter die Energie (E) der Komponente EA1 ist.

4. Vorrichtung nach Anspruch 1, wobei:
- der Bereich der AVD-Werte einen ersten Bereich mit einer Vielzahl von kurzen AVDs und einen zweiten Bereich, der sich von dem ersten unterscheidet, mit einer Vielzahl von langen AVDs umfasst;
- die Quantifizierungsmittel geeignet sind, den Variationsgrad durch Berechnen einer Differenz (Δ_{PEA1}) zwischen den Werten des ventrikulären EA-Parameters, die für die AVD des ersten Bereichs aufgezeichnet wurden und jenen, die für den zweiten Bereich aufgezeichnet wurden, zu ermitteln; und
- die Mittel zum Erfassen der atrialen Aktivität geeignet sind, zwischen einer normalen atrialen mechanischen Aktivität, wenn die Differenz größer ist als ein vorbestimmter Schwellenwert, und im entgegengesetzten Fall einer fehlenden oder mangelhaften atrialen mechanischen Aktivität, zu unterscheiden.

5. Vorrichtung nach Anspruch 1, wobei:
- die Quantifizierungsmittel geeignet sind, den Variationsgrad durch Berechnen einer Standardabweichung (SD_{PEA1}) der aufgezeichneten Werte des ventrikulären EA-Parameters für die verschiedenen AVDs des Wertebereichs zu ermitteln; und
- die Mittel zum Erfassen der atrialen Aktivität geeignet sind, zwischen einer normalen atrialen mechanischen Aktivität, wenn die Standardabweichung größer ist als ein vorbestimmter Schwellenwert, und im entgegengesetzten Fall einer fehlenden oder mangelhaften atrialen mechanischen Aktivität, zu unterscheiden.

6. Vorrichtung nach Anspruch 1, wobei:
- die Quantifizierungsmittel geeignet sind, den Variationsgrad durch Modellieren der sigmoiden Charakteristik (MS) der Variation des ventrikulären EA-Parameters in Abhängigkeit von den AVDs des Wertebereichs mit zwei Plateaus (P1, P2) beiderseits eines mittleren Übergangsabschnitts zu ermitteln; und
- die Mittel zum Erfassen der atrialen Aktivität geeignet sind, zwischen einer normalen atrialen mechanischen Aktivität, wenn die absolute Niveaudifferenz (ΔP) der beiden Plateaus größer ist als ein vorbestimmter Schwellenwert, und im entgegengesetzten Fall einer fehlenden oder mangelhaften atrialen mechanischen Aktivität, zu unterscheiden.

7. Vorrichtung nach Anspruch 1, umfassend ferner Mittel zum Speichern eines Indexes abhängig vom Variationsgrad des ventrikulären EA-Parameters in Abhängigkeit von der Vielzahl von Werten der AVD, und Mittel zur Bildung einer Chronologie der Entwicklung des Indexes im Zeitverlauf.

8. Vorrichtung nach Anspruch 7, ferner umfassend:
- Mittel zur atrialen Stimulation; und
- Mittel, die geeignet sind: die Mittel zum Erfassen der atrialen Aktivität in Gegenwart mindestens eines spontanen atrialen Ereignisses und mindestens eines stimulierten atrialen Ereignisses einzusetzen; die gespeicherten jeweiligen Werte des Indexes zu vergleichen; und, im Fall einer Diskrepanz zwischen den jeweiligen verglichenen Indices, aus diesem Vergleich einen Warnindikator abzuleiten.

9. Vorrichtung nach Anspruch 7, ferner umfassend Mittel, die geeignet sind, im Fall des Eintretens einer Episode einer atrialen Fibrillation (AF), die gespeicherten, jeweils der atrialen Fibrillationsepisode vorausgehenden und nachfolgenden Werte des Indexes zu vergleichen und aus diesem Vergleich einen Erholungsindikator oder einen Indikator für die Abwesenheit von Erholung nach der atrialen Fibrillation abzuleiten.

## Claims

1. An active implantable medical device such as a cardiac prosthesis for stimulation, resynchronization and/or defibrillation, comprising:
- means for detecting atrial and ventricular events;
- means for ventricular stimulation;
- means for applying to the stimulation means an atrioventricular delay, AVD, counted from the detection of a spontaneous or stimulated atrial event and at the end of which a ventricular stimulation is delivered in the absence of detection of a corresponding spontaneous ventricular event;
- an acceleration sensor adapted to deliver an endocardiac acceleration, EA, signal representative of the movements produced by the cyclic contractions of the myocardium;
- means for analysing the EA signal, adapted to derive from this EA signal a value of a non-time EA ventricular parameter representative of a component EA1 of the signal corresponding to the first EA peak associated with the isovolumic ventricular contraction;
- scanning means, adapted to vary the DAV in a controlled manner in a range comprising a plurality of DAV values; and
- quantification means, to evaluate a degree of variation of said EA ventricular parameter as a function of said plurality of DAV values,
**characterized in that** it further comprises:
- atrial activity detection means, adapted to discriminate between:
. a normal atrial mechanical activity, and
. an absent or deficient atrial mechanical activity despite an electrical activity observable in sinusal rhythm,
as a function of said degree of variation evaluated by the quantification means.

2. The device of claim 1, wherein said non-time EA ventricular parameter is the amplitude between extrema (PEA1) of the EA signal of the component EA1.

3. The device of claim 1, wherein said non-time EA ventricular parameter is the energy (E) of the component EA1.

4. The device of claim 1, wherein:
- said range of DAV values comprises a first range with a plurality of short DAVs and a second range, distinct from the first one, with a plurality of long DAVs;
- the quantification means are adapted to evaluate said degree of variation by calculation of a difference (Δ_{PEA1}) between the values of the EA ventricular parameter measured for the DAVs of the first range and those measured for the second range; and
- the atrial activity detection means are adapted to discriminate between a normal atrial mechanical activity when said difference is higher than a predetermined threshold, and an absent or deficient atrial mechanical activity in the opposite case.

5. The device of claim 1, wherein:
- the quantification means are adapted to evaluate said degree of variation by calculation of a standard deviation (SD_{PEA1}) of the values of the EA ventricular parameter measured for the various DAVs of said range of values; and
- the atrial activity detection means are adapted to discriminate between a normal atrial mechanical activity when said standard deviation is higher than a predetermined threshold, and an absent or deficient atrial mechanical activity in the opposite case.

6. The device of claim 1, wherein:
- the quantification means are adapted to evaluate said degree of variation by modelling of the sigmoid characteristic (MS) of variation of the EA ventricular parameter as a function the DAVs of said range of values, with two stationary phases (P1, P2) on either side of central transition portion; and
- the atrial activity detection means are adapted to discriminate between a normal atrial mechanical activity when the absolute difference of level (ΔP) of the two stationary phases is higher than a predetermined threshold, and an absent or deficient atrial mechanical activity in the opposite case.

7. The device of claim 1, further comprising means for memorizing an index that is function of said degree of variation of the EA ventricular parameter as a function of the plurality of DAV values, and means for constituting an history of the evolution of said index over time.

8. The device of claim 7, further comprising:
- atrial stimulation means; and
- means adapted to: implement said atrial activity detection means in the presence of at least one spontaneous atrial event and at least one stimulated atrial event; compare the respective memorized values of said index; and derive from this comparison an alert indicator in case of discordance between the respective indices compared.

9. The device of claim 7, further comprising means adapted, in case of occurrence of an atrial fibrillation (AF) episode, to compare the memorized values of said index respectively before and after the episode of atrial fibrillation, and to derive from this comparison an indicator of recovery, or of absence of recovery, after atrial fibrillation.
